# NEUE EUROPÄISCHE PATENTSCHRIFT

(11) **EP 0 347 751 B2**
(45) Veröffentlichungstag und Bekanntmachung des Hinweises auf die Entscheidung über den Einspruch: **16.06.1999**
(45) Hinweis auf die Patenterteilung: 31.03.1993
(21) Anmeldenummer: 89110871.4
(22) Anmeldetag: 15.06.1989
(51) Int. Cl.: A61K 9/50

(54) **Stabile, kaltwasserdispergierbare Präparate**
Stable compositions dispersible in cold water
Compositions stables et dispersables dans l'eau froide

(30) Priorität: 23.06.1988 CH 241688; 17.04.1989 CH 144489
(43) Veröffentlichungstag der Anmeldung: 27.12.1989
(73) Patentinhaber: F. HOFFMANN-LA ROCHE AG, 4002 Basel (CH)
(72) Erfinder: Berneis, Kurt, Dr., CH-4107 Ettingen (CH); Schuler, Peter, CH-4056 Basel (CH)
(74) Vertreter: Cottong, Norbert A.

(56) Entgegenhaltungen:
- EP-A- 0 346 879
- GB-A- 1 200 906
- US-A- 4 670 247
- CHEMICAL ABSTRACTS, Band 102, Nr. 16, 22. April 1985, Seite 379, Zusammenfassung Nr. 137846z, Columbus, Ohio, US
- CHEMICAL ABSTRACTS, Band 108, Nr. 8, 22. Februar 1988, Seite 658, Zusammenfassung Nr. 65887n, Columbus, Ohio, US; NORLAND PRODUCTS, INC.: "Small crystal formation using fish gelatin", & RES. DISCL. 1987, 284, 788
- Mikroinkapslet foder til marine fiskelarver, Kim Jacobsen, 1987, Forschungsproject, Novo Industri A/S & Fisheries Laboratory of the danish University of technology und eine entsprechende englische Ubersetzung dieses Dokuments
- Datenblatt Hipure Liquid Gelatin
- Datenblatt Vitamin A-acetat CWD
- Datenblatt Vitamin A-palmitat CWD
- Datenblatt Vitamin E-acetat Trockenpulver coated 25 CWD
- Datenblatt Gelita-SOL
- J. Pharm. Pharmac., 20, 1968, 348-354

## Beschreibung

Die vorliegende Erfindung betrifft neue, stabile, kaltwasser-dispergierbare Präparate von fettlöslichen Substanzen, ein Verfahren zu deren Herstellung sowie die Verwendung von Fischgelatine zur Herstellung solcher Präparate.

Der Ausdruck "kaltwasser-dispergierbare Präparate'' bedeutet im Rahmen der vorliegenden Erfindung, feste Anwendungsformen, d.h. in Pulverform vorliegende Präparate. Der Ausdruck "fettlösliche Substanzen" umfasst im Rahmen der vorliegenden Erfindung insbesondere die fettlöslichen Vitamine A, D, E und K, Carotinoide wie z.B. Beta-Carotin, Astaxanthin, Apocarotenal, Canthaxanthin, Zeaxanthin usw., sowie auch mehrfach ungesättigte (polyungesättigte) Fettsäuren und dergleichen. Es sind jedoch ohne weiteres auch andere fettlösliche Substanzen denkbar, welche in der menschlichen oder tierischen Ernährung eine Rolle spielen und wie die vorhergehend genannten in Folge ihrer Wasserunlöslichkeit oder auch ihrer mehr oder weniger ausgeprägten Stabilität und Handhabbarkeit in der Regel in Form von Emulsionen oder Trokkenpulvern im Handel sind. Hier können insbesondere genannt werden Oele und Fette, wie z.B. Sonnenblumenöl, Palmöl. Rinderfett und dergleichen. Gemeinsam ist derartigen Präparaten in der Regel, dass die Wirkstoffe (die fettlöslichen Substanzen) mittels eines Schutzkolloides umhüllt sind, welches dann u.a. für den Schutz des Wirkstoffes bzw. für dessen Stabilisierung, für eine optimale Resorption und für die allfällig nötige Wasserdispergierbarkeit des Endpräparates verantwortlich ist. Als Schutzkolloid wird häufig Gelatine verwendet, welche von Warmblütern stammt und demgemäss auch gewisse Nachteile aufweist. Als Beispiele seien hier nur genannt die Tatsache, dass etwa Präparate auf der Basis derartiger Gelatine aus religiösen Gründen nicht weltweit Verwendung finden können, dass diese Gelatine, ohne aufwendige Herstellungsver-fahren, und demgemäss auch die damit hergestellten. pulverförmigen Präparate, nicht immer die gewünschte Kaltwasserdispergierbarkeit aufweisen usw.

Erfindungsgemäss hat sich nun herausgestellt, dass all diese Nachteile eliminiert werden können, wenn an Stelle von Gelatine von Warmblütern Fischgelatine verwendet wird.

Die erfindungsgemässen, stabilen, kaltwasserdispergierbaren. pulverförmigen, mit einem Schutzkolloid stabilisierten bzw. umhüllten Präparate fettlöslicher Substanzen sind dadurch gekennzeichnet, dass sie als Schutkolloid Fischgelatine enthaltend, aber nicht das Vernetzungsmittel Glutaraldehyd

Die UK-Patentschrift 1.200.906 beschreibt ein Verfahren zur Herstellung von fettlösliche Vitamine enthaltenden Präparaten, die in Form von trockenen, freifliessenden Partikeln und in kaltem Wasser löslich oder dispergierbar sind. Dieses Verfahren beinhaltet die partielle Hydrolyse einer Gelatine-Lösung durch Behandlung mit einer Base oder einer anorganischen Säure bei Temperaturen von 40° C bis 95°C, Neutralisieren der so erhaltenen Gelatine-Lösung auf pH 4.5-7.0 und feines Dispergieren des Vitamins oder Vitamingemisches in der Gelatine-Lösung, was zur Bildung einer Emulsion führt, die anschliessend in trockene, freifliessende Partikel übergeführt wird, z. B. durch Sprühtrocknung der Emulsion. Die in dieser Patentschrift beschriebene Erfindung beruht auf dem Befund, dass Gelatine, die einer partiellen Hydrolyse nicht unterworfen worden ist, nicht nachträglich. d.h. bei deren Anwendung, bei normalen (nicht erhöhten) Anwendungstemperaturen in Wasser gelöst oder dispergiert werden kann. Ferner wird in diesem Verfahren eine Gelatine mit einer "hohen oder "niedrigen" Bloom-Zahl verwendet, wobei in den diesbezüglichen Beispielen diese Zahl entweder 130 oder 70 beträgt. Von der Verwendung von Fischgelatine ist in dieser Patentschrift keine Rede, und alle Hinweise (insbesondere die Bloom-Zahl) deuten klar und eindeutig darauf hin, dass es sich bei der verwendeten Gelatine um ganz normale Gelatine aus tierischen Quellen handelt, Das Gleiche gilt auch für den in dieser Patentschrift berichteten Stand der Technik.

Die US-Patentschrift 4.670.247 beschreibt ebenfalls fettlösliche Vitamine und Gelatine enthaltende Präparate, die allerdings wasserunlöslich sind. Solche Präparate, die in Form von Kügelchen (beadlets) vorliegen, werden dadurch hergestellt. dass man eine Emulsion aus einem Vitamin, Wasser. Gelatine sowie einem Zucker bildet, diese in Tröpfchen überführt und anschliessend in einer Masse Stärkepulver sammelt, die so gebildeten einzelnen, Vitamine enthaltenden Partikel vom noch verbleibenden, überflüssigen Stärkepulver trennt und die Partikel bei einer Temperatur von ca. 90° C bis ca. 180°C erhitzt. Erwähnt wird u.a., dass Gelatine mit einer Bloom-Zahl von ca. Null bis ca. 300 im Herstellungsverfahren verwendet werden kann, und zwar sowohl "Type A"-Gelatine wie auch "Type B"-Gelatine. Wiederum handelt es sich bei der zu verwendenden Gelatine klarerweise um Gelatine aus tierischen Quellen, da auch in den diesbezüglichen Beispielen die verwendete Gelatine eine Bloom-Zahl von 75, 100 bzw. 200 aufweist. Ein weiterer Hinweis, dass es sich bei der angesprochenen Gelatine stets um tierische Gelatine handelt, befindet sich in der Spalte 2, Zeilen 47 und 48, wo steht, dass die Gelatine in Wasser unter leichtem Erwärmen löst: Fischgelatine ist bereits in kaltem Wasser löslich und geliert nicht bei Raumtemperatur.

Gegenüber der Lehre aus beiden obenerwähnten Patentschriften handelt es sich bei den erfindungsgemässen Präparaten nicht bloss um mit Gelatine umhüllte, fettlösliche Substanzen, sondern vielmehr um Emulsionen der jeweiligen fettlöslichen Substanz mit Fischgelatine.

Bei der in der europäischen Patentpublikation 346 879 (Stand der Technik nach Art.54(3)EPÜ) beschriebenen Erfindung handelt es ich um durch Fischgelatine umhüllte, feste, wasserunlösliche Partikel von Medikamenten ("drugs"), deren unangenehmer Geruch und Geschmack durch das Vorhandensein des Umhüllmaterials maskiert oder eliminiert werden. Demzufolge werden die betreffenden Medikamente von den Patienten eher nach ärztlicher Weisung eingenommen. Im Gegensatz zu den erfindungsgemässen Präparaten werden die in dieser Patentpublikation beschriebenen umhüllten Medikamente nicht durch Emulgieren hergestellt, sondern durch Dispergieren der festen, zu umhüllenden Partikel in einer Lösung von Fischgelatine, insbesondere in Wasser, gefolgt von Koazervation der Fischgelatine durch Zugabe eines konventionellen Koazervierungsmittels. Die Koazervierungsmittel lassen die Fischgelatine um die festen Partikel (des Medikaments) anlagern, und zwar unter resultierender Bildung einer Suspension von derartigen umhüllten Partikeln im modifizierten, wässrigen Medium. Die anschliessende Zugabe eines fixierend-quervernetzenden Mittels, z.B. Glutaraldehyd, lässt die Gelatine fest und absolut wasserunlöslich werden. Alsdann können die umhüllten Partikel vom Medium entfernt werden, und zwar nach konventionellen Methoden, wie beispielsweise Ultrazentrifugation oder Filtration. Die Verwendung der Fischgelatine im bekannten Verfahren ermöglicht die Koazervierung bei relativ niedrigen Temperaturen, d.h. bei niedereren Temperaturen als diejenigen, bei denen die Koazervation mit Gelatine aus tierischen Quellen normalerweise erfolgt (siehe Seite 2, Spalte 1, Zeilen 29-40. sowie Spalte 2, Zeilen 2-5, der europäischen Patentpublikation).

Die erfindungsgemässen Präparate unterscheiden sich im wesentlichen von den in der Patentpublikation beschriebenen Präparaten nicht nur durch die Anwesenheit einer fettlöslichen Substanz gegenüber einem festen partikelförmigen wasserunlöslichen Medikament, sondern auch durch das grundsätzlich verschiedene Herstellungsverfahren. das den erfindungsgemässen Präparaten ganz andere physikalische Merkmale verleiht gegenüber den bekannten Präparaten. So zeichnen sich die in der europäischen Patentpublikation beschriebenen Produkte zwangsläufig durch die Anwesenheit eines Koazervierungsmittels sowie eines fixierendquervernetzenden Mittels aus. welche beide klarerweise im bekannten Herstellungsverfahren verwendet werden. Im Gegensatz dazu werden die erfindungsgemässen Präparate dadurch hergestellt, dass man zunächst einmal eine wässrige Emulsion des Wirkstoffes mit einem Schutzkolloid, nämlich Fischgelatine, vorbereitet, wobei letztere nicht nur als Schutzkolloid sondern auch als Emulgator dient. Obwohl bei der Herstellung der Emulsion auch weitere (normalerweise in derartigen Präparaten übliche) Hilfsstoffe und/oder Emulgatoren verwendet werden können, unterscheiden sich diese weitgehend von den in der Patentpublikation erwähnten Koazervierungsmitteln und fixierend-quervernetzenden Mitteln.Das Emulgieren stellt bei der vorliegenden Erfindung ein sehr wichtiges Merkmal dar. Infolge des ganz besonderen und einzigartigen Herstellungsverfahrens weisen die erfindungsgemässen Präparate selbst eine Emulsion der jeweiligen fettlöslichen Substanz mit dem Schutzkolloid Fischgelatine auf, was die stabile Dispergierbarkeit dieser Präparate in kaltem Wasser ermöglicht. Die bekannten Präparate sind hingegen nicht oder nur leicht wasserdispergierbar.

Wie oben erwähnt, können die erfindungsgemässen Präparate dadurch hergestellt werden, dass man eine wässrige Emulsion der fettlöslichen Substanz und des Schutzkolloids Fischgelatine herstellt und diese Emulsion anscnliessend in ein Trockenpulver überführt. Dies stellt das erfindunsgemässe Verfahren dar.

Die im Rahmen der vorliegenden Erfindung verwendete Fischgelatine kann im Prinzip in zur Gelatine von Warmblütern analoger Weise hergestellt werden, jedoch werden hier ausschliesslich Fischhäute verwendet. Bevorzugt sind zudem Häute von Tiefseefischen, wie etwa Kabeljau, Schellfisch, Dorsch usw. Eine derartige Fischgelatine hat einen Gelierpunkt unterhalb ca. 20° C, insbesondere zwischen etwa 5° C und etwa 10° C; dies im Gegensatz zu Gelatine von Warmblütern, welche bei ca. 35°C geliert. Eine besonders bevorzugte Fischgelatine ist die unter dem Namen "Norland HiPure Liquid Gelatin" erhältliche Gelatine der Firma Norland Products Inc., 695 Joyce Kilmer Ave., New Brunswick, N.J., USA.

Wie bereits vorhergehend erwähnt, können die erfindungsgemässen Präparate in an sich bekannter Weise hergestellt werden. Dies erfolgt normalerweise durch Emulgieren des Wirkstoffes oder der Wirkstoffe. d.h. der fettlöslichen Substanz(en), in einer Matrix mit anschliessendem Trocknen der so erhaltenen Emulsion.

Bei der Herstellung der Emulsion können neben Fischgelatine, welche sowohl als Emulgator als auch als Schutzkolloid dient, selbstverständlich auch weitere normalerweise in derartigen Präparaten übliche Hilfsstoffe verwendet werden. Als Beispiele hiervon können genannt werden, Zucker wie etwa Saccharose, Zuckeralkohole, Stärkederivate wie Maltodextrin, Milchproteine wie etwa Natriumkaseinat oder auch pflanzliche Proteine wie z.B. Sojaprotein, Kartoffelprotein, Weizenprotein usw.

In der Regel werden zunächst, ausser dem Wirkstoff, alle Bestandteile in Wasser gelöst, wobei die sogenannte Matrix erhalten wird. In diese Matrix wird sodann der Wirkstoff, bzw. werden die Wirkstoffe hineinemulgiert. Die Herstellung der Emulsion kann in an sich bekannter Weise erfolgen, beispielsweise durch kräftiges Rühren oder auch mittels Ultraschall und dergleichen. Der Druck und die Temperatur sind bei diesem Vorgang keine kritischen Parameter, und das Ganze kann ohne weiteres bei Temperaturen von etwa Raumtemperatur bis etwa 70° C und Atmosphärendruck durchgeführt werden.

Das Verhältnis von Oelphasen (fettlösliche Substanzen) zu den im Endprodukt letztlich vorhandenen Begleitstoffen beträgt in der Regel von ca. 1 % : 99 % bis etwa 60 % zu ca. 40%. Die genauen Mengenverhältnisse sind abhängig vom jeweiligen biologischen Bedarf an Wirkstoffen und von der Forderung nach gleichmässiger und aussreichend feiner Verteilung der Endpräparate in den zur Konzumation vorgesehenen Anwendungsformen. Sollten auch noch stabilisierende Substanzen in den Präparaten nötig oder erwünscht sein, so können diese in der Regel in der Oelphase gelöst werden. Wie bereits erwähnt, dient bei der Herstellung der Ernulsion die Fischgelatine auch als Emulgator. Es können jedoch auch noch weitere Emulgatoren verwendet werden, wobei hier in erster Linie z.B. Ascorbylpalmitat in Frage kommt, welches dann zudem auch noch als Stabilisator wirkt.

Die Ueberführung einer so hergestellten Emulsion in Trockenpulver, kann ebenfalls in an sich bekannter Weise erfolgen, z.B. durch normale Sprühtrocknung, mittels Doppeldispergier-Verfahren oder auch mittels Stärke-Catch-Verfahren. Bei letzterern Verfahren werden die versprühten Emulsionströpfchen in einem Stärkebett aufgefangen und anschliessend der Trocknung zugeführt.

Die erfindungsgemässen Präparate können sowohl in der Tiernahrung als auch für menschliche Ernährung Verwendung finden.

Der Ausdruck "Fischgelatine" bedeutet in den nachfolgenden Beispielen jeweils die von der Firma Norland stammende "Norland HiPure Gelatin".

### Beispiel 1

In einem 600 ml Becherglas werden 144 g Fischgelatine (als ca. 45 %-ige wässrige Lösung) und 97.2 g Kristallzucker vorgelegt. Dann werden 20 ml destilliertes Wasser zugesetzt und das Ganze unter Rühren mit einem Flügelrührer (2800 U/Min.) bei 40° C in Lösung gebracht. Hierauf werden 100 g Vitamin A-Palmitat (1,7 Millionen IE g und stabilisiert mit α-Tocopherol) in diese Matrix hineinemulgiert und während 60 Minuten weitergerührt. Nach dieser Zeit weist die innere Phase der Emulsion eine mittlere Teilchengrösse von etwa 0.6 µ auf. Die Emulsion wird dann mit 100 ml destilliertem Wasser verdünnt und auf 65° C aufgewärmt. In einer Laborsprühwanne werden dann ca. 1 Kg mittels Kieselsäure fluidisierte Stärke vorgelegt und auf ca. 5° C gekühlt. Hierein wird nun die Emulsion mittels einer rotierenden Sprühdüse eingesprüht. Die so erhaltenen, mit Stärke umhüllten Partikel werden dann von der überschüssigen Stärke abgesiebt und bei Raumtemperatur mittels Pressluft getrocknet. Man erhält ca. 330 g Trockenpulver mit einem Vitamin A Gehalt von 530'000 IE/g.

### Beispiel 2

In zu Beispiel 1 analoger Weise, wird ausgehend von 117 g Fischgelatine (als ca. 45%-ige wassrige Lösung), 58,4 g Kristallzucker, 20 ml cestilliertem Wasser und 13,5 g Vitamin A-Palmitat (1,7 Millionen IE/g, stabilisiert mit α-Tocoperol) eine Emulsion hergestellt. Diese Emulsion wird mit 70 ml Wasser verdünnt. Der mittlere Teilchendurchmesser der inneren Phase beträgt etwa 0,3 µ. Nach dem Trocknen erhält man 160 g Trockenpulver mit einem Vitamin A-Gehalt von 139'600 IE/g.

### Beispiel 3

In zu Beispiel 1 analoger Weise, wird ausgehend von 51,2 g Fischgelatine (als Trockensubstanz), 76,8 g Maltodextrin MDO5 (der Firma Roquettes Frères, in Lille, Frankreich), 80 ml destilliertes Wasser, 31,9 g einer öligen Lösung von 25 g Vitamin A-Acetat (2,8 Millionen IE/g) und 2.5 g α-Tocopherol in 4,4 g Arachisöl eine Emulsion hergestellt. Diese Emulsion wird mit 90 ml destilliertem Wasser verdünnt, Der mittlere Teilchendurchmesser der inneren Phase beträgt 0.28 µ. Nach dem Trocknungsvorgang erhält man 195 g Trockenpulver mit einem Vitamin A-Gehalt von 351 300 IE g.

### Beispiel 4

In zu Beispiel 1 analoger Weise, wird aus 31,3 g Fischgelatine (als ca. 45%-ige wässrige Lösung), 42,3 g Maltodextrin MD05 (der Firma Roquettes Frères, in Lille, Frankreich), 20 ml destilliertem Wasser und 63,6 g Tocopherolacetat eine Emulsion hergestellt. Diese Emulsion wird mit 200 ml Wasser verdünnt. Der mittlere Teilchendurchmesser der inneren Phase beträgt 0,34 µ. Diese Emulsion wird dann in einem Laborsprühtrockner der Firma Büchi in Flawil, Schweiz, sprühgetrocknet. Die Eintrittstemperatur beträgt 186° C und die Austrittstemperatur 106° C. Man erhält so 115 g Trockenpulver mit einem Tocopherolacetat-Gehalt von 52,1 %.

### Beispiel 5

In zu Beispiel 1 analoger Weise wird aus 28,5 g Fischgelatine (als Trockensubstanz), 42,7 g Maltodextrin MD05 (der Firma Roquettes Frères, in Lille, Frankreich), 50 ml destilliertem Wasser und 84,8 g einer öligen Lösung von 84 g Gamma-Linolensäure (als Triglycerid) und 0,8 g α-Tocopherol eine Emulsion hergestellt. Diese Emulsion wird mit 85 ml Wasser verdünnt. Der mittlere Teilchendurchmesser der inneren Phase beträgt 0,4 µ. Nach dem Trocknungsvorgang erhält man 200 g Trockenpulver mit einem Gehalt an Gamma-Linolensäure von 9,8 %.

### Beispiel 6

a) In einem 1 L Becherglas, werden 18 g Fischgelatine (als Trockensubstanz), 27 g Maltodextin MD05 (der Firma Roquettes Frères, in Lille, Frankreich) und 14,7 g Kristallzucker in 180 ml destilliertem Wasser bei 70° C gelöst. Zu der Lösung werden dann unter Rühren 5 g Ascorbylpalmitat gegeben und der pH der Lösung wird mittels 20%-iger Natronlauge auf 7,5 ± 0,2 eingestellt.
b) In einem 500 ml Rundkolben werden während 15 Minuten auf einem Dampfbad 13 g β-Carotin, 5,5 g Arachisöl und 1,5 g α-Tocopherol in 200 ml Chloroform gelöst.
c) In einem 2 L Rundkolben wird die gemäss b) erhaltene β-Carotin-Lösung während 30 Minuten bis 40° C in die gemäss a) hergestellte Lösung hineinemulgiert. Nach dieser Zeit weist die innere Phase eine Teilchengrösse von etwa 0,18 µ auf. Das Chloroform wird nun in einer Kurzwegdestillationsanlage bei 50° C am Wasserstrahlvakuum entfernt und die Emulsion in zu Beispiel 1 analoger Weise in Stärke versprüht. Man erhält 85 g Trokkenpulver mit einem β-Carotin-Gehalt von 12,5 %.

### Beispiel 7

In zu Beispiel 1 analoger Weise, wird ausgehend von 56,4 g Fischgelatine (als Trockensubstanz), 84,6 g Maltodextrin MD 05 (der Firma Roquettes Frères, Lille, Frankreich), 125 ml destilliertem Wasser und 159 g Sonnenblumenöl eine Emulsion hergestellt. Diese Emulsion wird mit 242 ml destilliertem Wasser verdünnt. Der mittlere Teilchendurchmesser der inneren Phase beträgt ca. 0,3 µ. Diese Emulsion wird dann in einem transportablen Minor Laborsprühtrockner der Firma NIRO Atomizer, Söborg, Dänemark, sprühgetrocknet. Die Eintrittstemperatur beträgt 200° C und die Austrittstemperatur
90-94° C, Man erhält so 230 g Trockenpulver mit einem Oelanteil von 53 %.

### Beispiel 8

In zu Beispiel 1 analoger Weise, wird ausgehend von 56,4 g Fischgelatine (als Trockensubstanz), 84,6 g Maltodextrin MD 05 (der Firma Roquettes Frères, Lille, Frankreich), 125 ml destilliertem Wasser und 159 g Rinderfett (stabilisiert mit 100-200 ppm Tocopherol) eine Emulsion hergestellt. Diese Emulsion wird mit 242 ml destilliertem Wasser verdünnt. Der mittlere Teilchendurchmesser der inneren Phase beträgt ca. 0,5 µ. Diese Emulsion wird dann in einem transportablen Minor Laborsprühtrockner der Firma NIRO Atomizer, Söborg, Dänemark, sprühgetrocknet. Die Eintrittstemperatur beträgt 200° C und die Austrittstemperatur 90-94° C. Man erhält so 235 g Trockenpulver mit einem Fettanteil von 53 %.

### Beispiel 9

In zu Beispiel 1 analoger Weise, wird ausgehend von 56,4 g Fischgelatine (als Trockensubstanz), 84,6 g Maltodextrin MD 05 (der Firma Roquettes Frères, Lille, Frankreich), 125 ml destilliertem Wasser und 159 g Palmöl eine Emulsion hergestellt. Diese Emulsion wird mit 242 ml destilliertem Wasser verdünnt. Der mittlere Teilchendurchmesser der inneren Phase beträgt ca. 0,3 µm. Diese Emulsion wird dann in einem transportablen Minor Laborsprühtrockner der Firma NIRO Atomzer Söborg, Dänemark, sprühgetrocknet. Die Eintrittstemperatur beträgt 200° C und die Austrittstemperatur 90-95° C. Man erhält so 225 g Trokkenpulver mit einem Oelanteil von 53 %.

## Patentansprüche

1. Stabile, kaltwasser-dispergierbare pulverförmige, mit einem Schutzkolloid stabilisierte bzw. umhüllte Präparate fettlöslicher Substanzen, dadurch gekennzeichnet, dass sie als Schutzkolloid Fischgelatine, aber nicht das Vernetzungsmittel Glutaraldehyd enthalten.

2. Präparate gemäss Anspruch 1, dadurch gekennzeichnet, dass sie als fettlösliche Substanzen die Vitamine A, D, E oder K oder ein Carotinoid oder eine polyungesättigte Fettsäure enthalten.

3. Präparate gemäss Anspruch 1, dadurch gekennzeichnet, dass sie als fettlösliche Substanzen Oele oder Fette, insbesondere Sonnenblumenöl, Palmöl oder Rinderfett, enthalten.

4. Verfahren zur Herstellung von Präparaten gemäss einem der Ansprüche 1 bis 3, dadurch gekennzeichnet, dass man eine wässrige Emulsion der fettlöslichen Substanz und des Schutzkolloids Fischgelatine herstellt und diese Emulsion anschliessend in ein Trockenpulver überführt.

5. Verwendung von Fischgelatine zur Herstellung von stabilen, kaltwasserdispergierbaren pulverförmigen Präparaten fettlöslicher Substanzen in der Abwesenheit von Glutaraldehyd.

## Claims

1. Stable, cold water-dispersible, pulverous preparations of fat-soluble substances which are stabilized or enveloped with a protective colloid, characterized in that they contain fish gelatine as the protective colloid but not glutaraldehyde as fixing crosslinking agent.

2. Preparations in accordance with claim 1, characterized in that they contain vitamin A, D, E or K or a carotinoid or a polyunsaturated fatty acid as the fat-soluble substance.

3. Preparations in accordance with claim 1 which contain oils or fats, especially sunflower oil, palm oil or beef fat. as the fat-soluble substance.

4. A process for the manufacture of preparations in accordance with any one of claims 1 to 3, characterized by preparing an aqueous emulsion of the fat-soluble substance and the fish gelatine protective colloid and, subsequently converting this emulsion into a dry powder.

5. The use of fish gelatine for the manufacture of stable cold water-dispersible, pulverous preparations of fat-soluble substances in the absence of glutaraldehyde.

## Revendications

1. Compositions de substances liposolubles, pulvérulentes, stables, dispersables à l'eau froide, stabilisées ou enrobées par un colloïde protecteur, caractérisées en ce qu'elles contiennent en tant que colloïde protecteur de la gélatine de poisson mais pas en tant qu'agent fixateur-réticulant l'aldéhyde glutarique.

2. Compositions selon la revendication 1, caractérisé en ce qu'elles contiennent en tant que substances liposolubles les vitamines A, D, E ou K ou un caroténoide ou un acide gras polyinsaturé.

3. Compositions selon la revendication 1, caractérisé en ce qu'elles contiennent en tant que substances liposolubles des huiles ou des graisses, en particulier de l'huile de tournesol, de l'huile de palme ou de la graisse de boeuf.

4. Procédé de préparation de compositions selon l'une des revendications 1 à 3, caractérisé en ce que l'on prépare une émulsion aqueuse de la substance liposoluble et de la gélatine de poisson servant de colloïde protecteur et on convertit ensuite cette émulsion en poudre sèche.

5. Utilisation de gélatine de poisson pour la préparation de compositions de substances liposolubles, pulvérulentes, stables et dispersables à l'eau froide en l'absence de l'aldéhyde glutarique.
